# FASCICULE DE BREVET EUROPEEN

(11) **EP 4 154 263 B1**
(45) Date de publication et mention de la délivrance du brevet: **27.05.2026**
(21) Numéro de dépôt: 21733488.7
(22) Date de dépôt: 20.05.2021
(51) Int. Cl.: G16H 30/40

(54) **MÉTHODE DE DÉTERMINATION D'UNE RÉGION D'ABLATION BASÉE SUR L'APPRENTISSAGE PROFOND**
VERFAHREN ZUR BESTIMMUNG EINES ABLATIONSBEREICHS AUF BASIS VON TIEFENLERNEN
METHOD FOR DETERMINING AN ABLATION REGION BASED ON DEEP LEARNING

(30) Priorité: 20.05.2020 FR 2005340
(43) Date de publication de la demande: 29.03.2023
(73) Titulaire: Quantum Surgical, 34000 Montpellier (FR)
(72) Inventeur: OUBEL, Estanislao, 34090 Montpellier (FR); BLONDEL, Lucien, 34070 Montpellier (FR); NAHUM, Bertin, 34170 Castelnau-Le-Lez (FR); BADANO, Fernand, 69006 Lyon (FR); GIRARDOT, Michael, 34170 Castelnau-Le-Lez (FR)
(74) Mandataire: Santarelli
(86) Numéro de dépôt international: PCT/FR2021/050907
(87) Numéro de publication internationale: WO 2021/234305

(56) Documents cités:
- ZHANG SIYUAN ET AL: "Detection and Monitoring of Thermal Lesions Induced by Microwave Ablation Using Ultrasound Imaging and Convolutional Neural Networks", IEEE JOURNAL OF BIOMEDICAL AND HEALTH INFORMATICS, IEEE, PISCATAWAY, NJ, USA, vol. 24, no. 4, 5 September 2019 (2019-09-05), pages 965 - 973, XP011781554, ISSN: 2168-2194, [retrieved on 20200402], DOI: 10.1109/JBHI.2019.2939810
- EGGER JAN ET AL: "Interactive Volumetry Of Liver Ablation Zones", vol. 5, no. 1, 20 October 2015 (2015-10-20), XP055772148, Retrieved from the Internet <URL:http://www.nature.com/articles/srep15373> [retrieved on 20200203], DOI: 10.1038/srep15373

## Description

### DOMAINE TECHNIQUE DE L'INVENTION

Le domaine de l'invention est celui de l'évaluation d'une intervention médicale.

Plus précisément, l'invention concerne une méthode d'évaluation post-traitement d'une région d'ablation d'une lésion et prédiction d'un risque de récidive associé.

L'invention trouve notamment des applications pour évaluer une intervention médicale mini-invasive en prédisant un risque de récidive d'une lésion, telle qu'une tumeur ou une métastase. Une telle intervention médicale mini-invasive correspond par exemple à une ablation percutanée de lésion, par exemple une tumeur dans le foie, un poumon, un rein ou tout autre organe. Une ablation percutanée consiste généralement à guider par l'image l'insertion d'une ou plusieurs aiguilles à travers la peau pour atteindre et détruire une lésion.

### ÉTAT DE LA TECHNIQUE

Il est connu de l'art antérieur des techniques permettant d'évaluer l'efficacité d'une intervention et de prédire un risque de récidive d'une lésion.

Une telle technique consiste par exemple suite à l'ablation d'une lésion, à déterminer dans quelle mesure la région d'ablation recouvre la lésion. En comparant le volume de la région d'ablation au volume de la lésion, il est possible de déterminer les marges d'ablation. En pratique, il est généralement recommandé d'avoir des marges d'ablation d'au moins cinq millimètres.

Afin de déterminer ces marges, le volume de la lésion est généralement déterminé lors de la planification de l'intervention et comparé au volume de la région d'ablation qui est segmenté par un opérateur sur au moins une image post-opératoire.

L'inconvénient majeur est que le volume de la région d'ablation est généralement déterminé avec peu de précision, d'une manière dépendant souvent de l'opérateur ayant effectué la segmentation. En outre, la qualité des images post-opératoires est souvent mauvaise, ce qui contribue à introduire des incertitudes dans la segmentation. Par conséquent, une corrélation entre des marges d'ablation et un risque de récidive d'une lésion est difficile à établir.

Afin d'améliorer les techniques de l'art antérieur, il est connu d'utiliser des méthodes de segmentation automatique de la région d'ablation.

Une telle technique est par exemple décrite dans la publication scientifique de Zhang et al, intitulée « Detection and Monitoring of Thermal Lesions Induced by Microwave Ablation Using Ultrasound Imaging and Convolutional Neural Networks », paru en septembre 2019. La méthode de segmentation décrite dans cette publication permet de calculer les marges de la région d'ablation en segmentant une image échographique pré-opératoire et une image échographique post-opératoire.

Toutefois, la méthode de segmentation décrite dans cette publication ne permet pas d'effectuer de prédiction du risque de récidive car la précision de la segmentation automatique de la région d'ablation est faible. D'une part la méthode de segmentation est limitée à une matrice de sous-échantillonnage d'une image de la région d'ablation, dont la taille est fixe, généralement égale à 4 mm², limitant ainsi l'utilisation de la méthode à des régions de dimensions réduites. En outre, il est nécessaire de connaître la position de la région d'ablation pour fixer la position de la matrice de sous-échantillonnage, rendant la méthode difficile à utiliser en l'absence de points de référence constants à l'intérieur de la matrice de sous-échantillonnage dans les images pré-opératoire et post-opératoire. Enfin, en raison de la nature et de la qualité des images échographiques en deux dimensions, l'anatomie d'intérêt peut être difficile à cerner, rendant la segmentation de la région peu précise, aboutissant notamment à des observations erronées où la région d'ablation telle que segmentée n'englobe pas la lésion objet de l'ablation.

En outre, les méthodes de segmentation automatique donnent des résultats cohérents pour des régions homogènes, c'est-à-dire par exemple pour un os, des vaisseaux sanguins, ou une lésion, ou lorsque l'image comprend un nombre connu de régions. Dans le cas de région d'ablations, les résultats de segmentation obtenus sont peu cohérents car les régions d'ablation sont des régions très complexes composées généralement de différentes matières telles que du gaz, des cellules nécrosées, des cellules saines, du produit de contraste résiduel, de la calcification, etc. Par ailleurs, la segmentation est généralement effectuée sur des images médicales généralement floues et peu contrastées, rendant la segmentation automatique de l'image difficile.

La publication scientifique "Interactive Volumetry Of Liver Ablation Zones", Egger Jan et al., vol. 5, no. 1 20 octobre 2015 (2015-10-20), XP055772148,DOI: 10.1038/ srep15373, concerne une méthode d'analyse d'une tumeur récurrente en effectuant un suivi dans le temps de l'altération des tissus autour de la région d'ablation de la tumeur.

Aucun des systèmes actuels ne permet de répondre simultanément à tous les besoins requis, à savoir de proposer une technique permettant d'évaluer finement un traitement d'ablation en segmentant une région d'ablation, notamment hétérogène, avec une meilleure précision, notamment à partir d'une image médicale peu nette et/ou peu contrastée.

### EXPOSÉ DE L'INVENTION

La présente invention vise à remédier à tout ou partie des inconvénients de l'état de la technique cités ci-dessus.

À cet effet, l'invention vise un procédé d'évaluation post-traitement d'une ablation d'une partie d'une anatomie d'intérêt d'un individu, l'anatomie d'intérêt comprenant au moins une lésion.

L'ablation est de type percutanée ou mini-invasive, ce qui consiste généralement à l'insertion d'au moins une aiguille à travers la peau pour atteindre et détruire une lésion. Plusieurs techniques d'ablation sont possibles : radiofréquence, micro-ondes, électroporation, laser, cryothérapie, ultrasons, etc.

L'anatomie d'intérêt peut être un foie, un poumon, un rein ou tout autre organe susceptible d'avoir une lésion.

Selon l'invention, le procédé d'évaluation post-traitement comprend des étapes de :
- acquisition d'une image médicale post-opératoire de l'anatomie d'intérêt de l'individu ;
- détermination automatique d'un contour de la région d'ablation par une méthode d'apprentissage automatique, de type réseau de neurones, analysant l'image post-traitement de l'anatomie d'intérêt de l'individu, ladite méthode d'apprentissage automatique étant préalablement entraînée lors d'une phase dite d'entraînement sur une base de données comportant une pluralité d'images médicales d'une anatomie d'intérêt identique d'un ensemble de patients, chaque images médicales de la base de données étant associée à une région d'ablation de l'anatomie d'intérêt dudit patient.

Ainsi, la région d'ablation est segmentée automatiquement dans l'image médicale post-opératoire en se basant sur un apprentissage préalable d'une pluralité d'images médicales segmentées par au moins un opérateur, préférentiellement par plusieurs opérateurs. Il convient de souligner que cette segmentation automatique par le réseau de neurones permet de s'affranchir de la présence d'un opérateur expérimenté dans l'analyse d'images médicales. En outre, la segmentation obtenue par le réseau de neurones s'avère généralement plus précise, notamment dans le cas d'analyse d'images en trois dimensions. Enfin, la segmentation automatique par un tel procédé est également de meilleure qualité dans le cas d'une image ayant un contraste et/ou une netteté faible(s).

Dans des modes particuliers de mise en œuvre de l'invention, la phase d'entraînement comprend une étape préalable d'entraînement sur des images médicales d'une anatomie d'intérêt identique comprenant une lésion non ablatée.

Ainsi, le réseau de neurones segmente mieux la région d'ablation dans l'image médicale post-opératoire de l'individu. Cet effet surprenant peut s'expliquer par le fait de similarité de forme et de position entre la lésion et la région d'ablation. En outre, il convient de souligner que le nombre d'images médicales accessibles présentant une lésion non ablatée d'une anatomie d'intérêt donné est généralement plus élevé que le nombre d'images médicales accessibles acquises après l'ablation de la lésion.

Dans d'autres modes particuliers de mise en œuvre de l'invention, le procédé d'évaluation post-traitement comprend également une étape de recalage de l'image post-opératoire et d'une image médicale de l'anatomie d'intérêt de l'individu, acquise avant le traitement chirurgical, dite image médicale pré-opératoire, l'image médicale pré-opératoire et l'image médicale post-opératoire recalées formant un couple d'images médicales de l'anatomie d'intérêt de l'individu,

Ainsi, une analyse de la position de région d'ablation par rapport à la lésion peut être effectuée.

On entend par image médicale pré-opératoire une image médicale acquise avant le traitement par ablation et par image médicale post-opératoire une image médicale acquise après le traitement par ablation.

Dans des modes particuliers de mise en œuvre de l'invention, le procédé d'évaluation post-traitement comprend également une étape d'évaluation d'un risque de récidive en fonction d'une caractéristique relative entre la région d'ablation et la lésion, entre la région d'ablation et l'anatomie d'intérêt ou entre la lésion et l'anatomie d'intérêt.

Ainsi, le procédé d'évaluation post-traitement selon l'invention offre au personnel médical une meilleure vision du traitement par ablation appliqué à l'individu, en lui permettant d'évaluer la nécessité d'un traitement complémentaire si le risque de récidive est avéré.

Le risque de récidive prend généralement la forme d'une valeur binaire, pouvant être égale par exemple à 0 ou à 1. On entendra par valeur positive lorsque le risque de récidive est avéré et par valeur négative lorsque le risque de récidive est faible.

Toutefois, le risque de récidive peut également prendre la forme d'une probabilité comprise entre 0 et 1. On entendra alors que le risque de récidive est avéré lorsque la valeur du risque est supérieure à une valeur seuil, par exemple égale à 0,5.

Dans des modes particuliers de mise en œuvre de l'invention, lorsque le risque de récidive est avéré, le procédé d'évaluation post-traitement comprend également une étape de détermination de la position de la récidive en fonction d'une caractéristique relative entre la région d'ablation et la lésion, entre la région d'ablation et l'anatomie d'intérêt ou entre la lésion et l'anatomie d'intérêt.

Dans des modes particuliers de mise en œuvre de l'invention, le risque de récidive est évalué en tenant compte d'une marge d'ablation entre la région d'ablation et la lésion.

Par exemple, les marges d'ablation sont égales ou supérieures à 5 mm pour que la valeur du risque de récidive soit négative.

La marge d'ablation est généralement définie comme étant la plus petite distance entre la région d'ablation et la lésion.

Dans des modes particuliers de mise en œuvre de l'invention, le risque de récidive est évalué en tenant compte d'une distance entre un centre de masse de la lésion et un centre de masse de la région d'ablation.

La valeur de référence du centre de masse dépend des marges d'ablation. Si les marges d'ablation sont égales à 10 mm et que la valeur de référence des marges d'ablation est de 5 mm, la distance entre les centres de masse de la lésion et les centres de masse de la région d'ablation doit être inférieure ou égale à 5 mm.

Dans des modes particuliers de mise en œuvre de l'invention, le risque de récidive est évalué en tenant compte de la régularité et de la netteté des bords de la région d'ablation par rapport au tissu sain environnant.

On entend par tissu sain environnant un tissu sain de l'anatomie d'intérêt situé à l'intérieur du cadre de l'image médicale recadrée.

Dans des modes particuliers de mise en œuvre de l'invention, le risque de récidive est évalué en tenant compte du rapport entre le volume de la lésion et le volume de la région d'ablation.

Dans des modes particuliers de mise en œuvre de l'invention, le risque de récidive est évalué en tenant compte d'une position de la lésion par rapport au centre de l'anatomie d'intérêt.

Dans des modes particuliers de mise en œuvre de l'invention, le procédé d'évaluation post-traitement comprend également une étape de segmentation de la lésion dans l'image médicale pré-opératoire de l'anatomie d'intérêt de l'individu.

Dans des modes particuliers de mise en œuvre de l'invention, le procédé d'évaluation post-traitement comprend également une étape de détection de la lésion dans l'image médicale pré-opératoire de l'anatomie d'intérêt de l'individu.

Dans des modes particuliers de mise en œuvre de l'invention, tout ou partie des images médicales de la base de données sont recadrés autour de la région d'ablation comprenant au moins une lésion, le recadrage des images étant effectué selon un cadre commun de dimension prédéterminée, l'ensemble des centres de la région d'ablation des images médicales recadrées de la base de données formant une constellation de points distincts à l'intérieur du cadre commun.

Ainsi, en répartissant la position des régions d'ablation dans le cadre commun, il est possible de réduire les erreurs de prédiction de la méthode d'apprentissage automatique. Dans le cas où l'ensemble des régions d'ablation seraient dans la même position dans le cadre commun, la méthode d'apprentissage automatique considérerait principalement les images post-opératoires ayant une région d'ablation dans cette position particulière, entraînant ainsi des erreurs de prédiction dans le cas où la région d'ablation serait dans une autre position.

Dans des modes particuliers de mise en œuvre de l'invention, pour l'ensemble des images médicales de la base de données, la partie du corps de l'individu comprise dans ladite image est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

Il convient de souligner que la répartition équivalente entre les unités élémentaires correspondant à une région d'ablation et les unités élémentaires à une région de non ablation peut être analysée au niveau d'une image ou globalement au niveau de l'ensemble des images.

Les unités élémentaires sont généralement appelés pixels dans le cadre d'images bidimensionnelles ou voxels dans le cadre d'images tridimensionnelles.

On entendra par parts quasiment égales lorsque les deux ensembles d'unités élémentaires sont constitués du même nombre d'unités élémentaires ou lorsque la différence de nombre d'unités élémentaires de chacun des deux ensembles est par exemple inférieure à 5 % du nombre d'unités élémentaires des deux ensembles.

Dans des modes particuliers de mise en œuvre de l'invention, la base de données d'images médicales post-opératoires comprend au moins une image médicale pré-opératoire comprenant au moins une lésion non ablatée.

Ainsi, la méthode d'apprentissage automatique est mieux entraînée.

Dans des modes particuliers de mise en œuvre de l'invention, le procédé d'évaluation post-traitement comprend également une étape de détermination d'un masque d'ablation complémentaire lorsque le risque de récidive est avéré.

Ainsi, une proposition de traitement est estimée dans un but d'éliminer le risque de récidive par le procédé d'évaluation post-traitement. Il convient de souligner que cette proposition de traitement est non contraignante, pouvant être suivie ou non par le personnel médical.

Dans des modes particuliers de mise en œuvre de l'invention, le procédé d'évaluation post-traitement comprend également une étape de planification d'une trajectoire d'un instrument médical jusqu'à un point cible d'une région d'ablation définie par le masque d'ablation complémentaire.

Il convient de souligner que cette étape de planification est effectuée préalablement au traitement complémentaire.

Dans des modes particuliers de mise en œuvre de l'invention, le procédé d'évaluation post-traitement comprend également une étape d'aide au suivi de la trajectoire planifiée par un opérateur de l'instrument médical.

Dans des modes particuliers de mise en œuvre de l'invention, la trajectoire planifiée et/ou d'une indication de guidage est affichée en temps réel sur un écran d'un dispositif de réalité augmentée.

Dans des modes particuliers de mise en œuvre de l'invention, les images médicales sont des images tridimensionnelles.

Il convient de souligner qu'une image tridimensionnelle peut correspondre à une collection d'images bidimensionnelles effectuées à intervalles généralement réguliers le long d'un axe prédéfini.

Dans des modes particuliers de mise en œuvre de l'invention, chaque image post-opératoire est acquise selon une même technique d'acquisition d'image.

En d'autres termes, la technique utilisée pour acquérir l'image médicale post-opératoire est identique à celle utilisée pour acquérir les images médicales post-opératoires de la base de données d'entrainement de la méthode d'apprentissage automatique.

L'invention concerne également un dispositif électronique comprenant un processeur et une mémoire informatique stockant des instructions d'un procédé selon l'un quelconque des modes de mise en œuvre précédents.

Un tel dispositif électronique peut être par exemple un dispositif de contrôle, un système de navigation, un dispositif robotisé ou un dispositif de réalité augmentée. Le dispositif de contrôle peut notamment être un ordinateur présent dans la salle d'opération ou un serveur distant.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
- la figure 1 est une vue schématique d'une intervention médicale ;
- la figure 2 est une vue synoptique d'un procédé d'évaluation post-traitement de l'intervention médicale de la figure 1 ;
- la figure 3 est un exemple d'image médicale tridimensionnelle dans laquelle une région d'ablation est mise en avant, utilisée au cours du procédé illustré en figure 2;
- la figure 4 est un exemple de quatre images médicales comprenant chacune un masque d'ablation délimité manuellement par un opérateur et un masque d'ablation prédit par un réseau de neurones du procédé illustré en figure 2 ;
- la figure 5 est un exemple d'image médicale dans laquelle une segmentation automatique d'une lésion et d'une région d'ablation est effectuée au cours d'une étape du procédé illustré en figure 2 ;
- la figure 6 illustre un exemple d'ablation complémentaire tel qu'éventuellement proposé par le procédé illustré en figure 2.

### DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

### Exemple d'un mode de mise en œuvre particulier

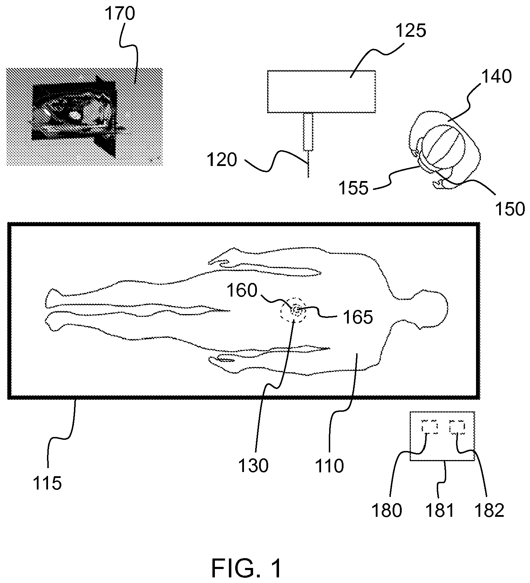

La figure 1 est une vue schématique d'une intervention médicale au cours de laquelle un individu 110 allongé sur une table 115 est traité à l'aide d'un instrument médical 120. Dans le présent exemple non limitatif de l'invention, l'intervention médicale correspond à l'ablation d'une lésion 165 dans une anatomie d'intérêt 130 qui est ici le foie de l'individu 110 par l'intermédiaire de l'instrument médical 120 qui est dans le cas présent une aiguille semi-rigide. L'intervention médicale est ici une procédure percutanée au cours de laquelle le corps de l'individu 110 n'est pas ouvert.

La manipulation de l'instrument médical 120 par un opérateur 140 peut être avantageusement guidée par l'intermédiaire d'un dispositif de guidage qui est dans le présent exemple non limitatif de l'invention un dispositif de réalité augmentée tel qu'un casque 150 porté par l'opérateur 140. L'instrument médical 120 peut également être associé à un dispositif 125 robotisé médical.

Le casque 150 comprend un écran 155 translucide permettant à l'opérateur de voir normalement. Sur l'écran 155, s'affiche en incrustation une image afin d'afficher des repères permettant de guider l'opérateur 140 dans la manipulation de l'instrument médical 120 en vue de traiter par ablation une région 160 dite d'ablation autour de la lésion 165 identifiée dans l'anatomie d'intérêt 130. Les repères peuvent notamment comprendre un masque d'ablation qui a été préalablement estimé sur une image médicale 170 de l'anatomie d'intérêt 130 acquise avant l'opération. L'image médicale 170 sera appelée par la suite image médicale pré-opératoire 170.

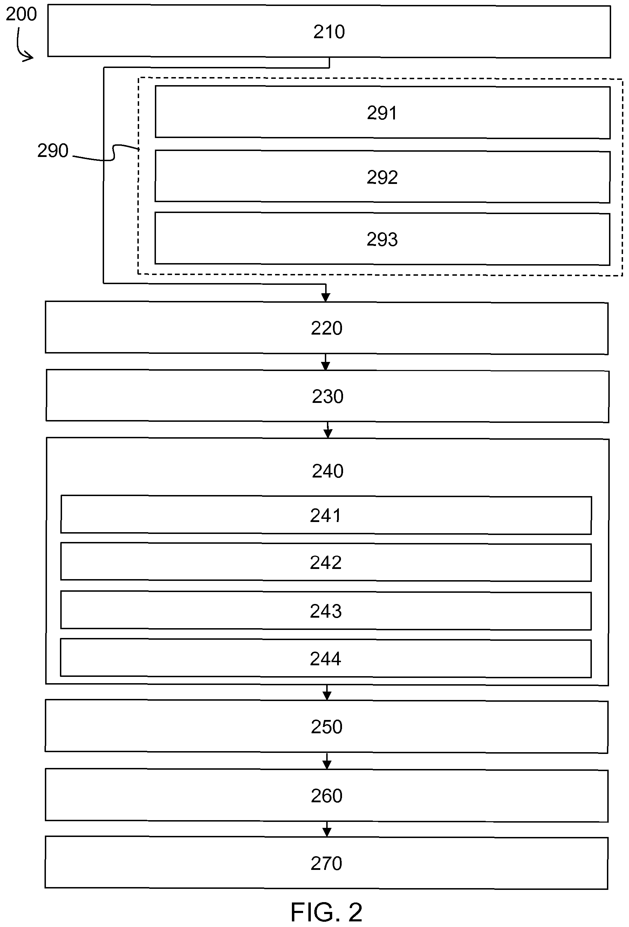

Lorsque l'opération est terminée, une évaluation du traitement opératoire est effectuée par un procédé 200 d'évaluation post-traitement de l'ablation tel qu'illustré sous forme synoptique en figure 2 et dont les instructions sont stockées dans une mémoire informatique 180 d'un dispositif électronique 181 de contrôle relié au casque 155, avec un câble ou par une technologie sans fil. Le procédé 200 d'évaluation post-traitement dont les instructions sont traitées par un processeur informatique 182 du dispositif électronique 181 de contrôle permet notamment de déterminer une région d'ablation et un risque de récidive associé, afin de vérifier si le traitement chirurgical effectué au cours de l'opération est suffisant ou s'il est préférable de poursuivre le traitement en effectuant par exemple une ablation complémentaire.

Il convient de souligner que le dispositif électronique 181 peut être avantageusement intégré dans le casque 150.

Le procédé 200 d'évaluation post-traitement comprend une première étape 210 d'acquisition d'une image médicale post-opératoire de l'anatomie d'intérêt 130.

Il convient de souligner que les images médicales pré-opératoire et post-opératoire sont de préférence acquises par tomodensitométrie. Alternativement elles peuvent être acquises par un appareil d'imagerie par résonance magnétique.

Préférentiellement, la technique utilisée pour acquérir l'image médicale pré-opératoire et l'image médicale post-opératoire est similaire, voire identique.

Toutefois, la technique utilisée pour acquérir l'image médicale post-opératoire peut être distincte de la technique utilisée pour acquérir l'image médicale pré-opératoire.

Les images médicales pré-opératoire et post-opératoire sont avantageusement dans le présent exemple non limitatif de l'invention des images acquises en trois dimensions. En pratique, chaque image médicale acquise en trois dimensions correspond généralement à une collection d'images médicales en deux dimensions correspondant chacune à une coupe de l'anatomie d'intérêt 130, effectuée à intervalles réguliers selon un axe prédéterminé. Une représentation tridimensionnelle de l'anatomie d'intérêt peut être reconstruite à partir de cette collection d'images médicales bidimensionnelles. On entendra ainsi par image en trois dimensions aussi bien une collection d'images médicales qu'une représentation tridimensionnelle. On entendra par voxel une unité élémentaire relative à la résolution de l'image en trois dimensions.

Alternativement, les images médicales pré-opératoire et post-opératoire sont acquises chacune en deux dimensions. L'unité élémentaire relative à la résolution de l'image en deux dimensions est alors couramment appelé pixel.

Les images médicales pré- et post-opératoire sont des images comprenant l'anatomie d'intérêt en entier ou recadrées autour de la région d'ablation selon un cadre prédéfini. Sur une image en trois dimensions, le cadre entourant la région d'ablation correspond à un cube. Tandis que sur une image en deux dimensions, le cadre correspond à un carré.

Le cadre entourant la région d'ablation, également connu sous le terme anglais « *bounding box* », peut être généré automatiquement autour de la région d'ablation suite à une action de l'opérateur. Une telle action peut par exemple correspondre au fait que l'opérateur indique un point dans l'image médicale post-opératoire appartenant à la région d'ablation et le cadre est généré autour de ce point. A titre d'exemple, dans le cadre d'un traitement d'ablation mini-invasive sur des lésions de petites tailles, c'est-à-dire par exemple sur des lésions de l'ordre de 5 cm +/- 10% de diamètre maximum, voire de préférence de l'ordre de 3 cm +/- 10% de diamètre maximum, chaque arête du cube ou chaque côté du carré mesure entre 5 et 10 cm.

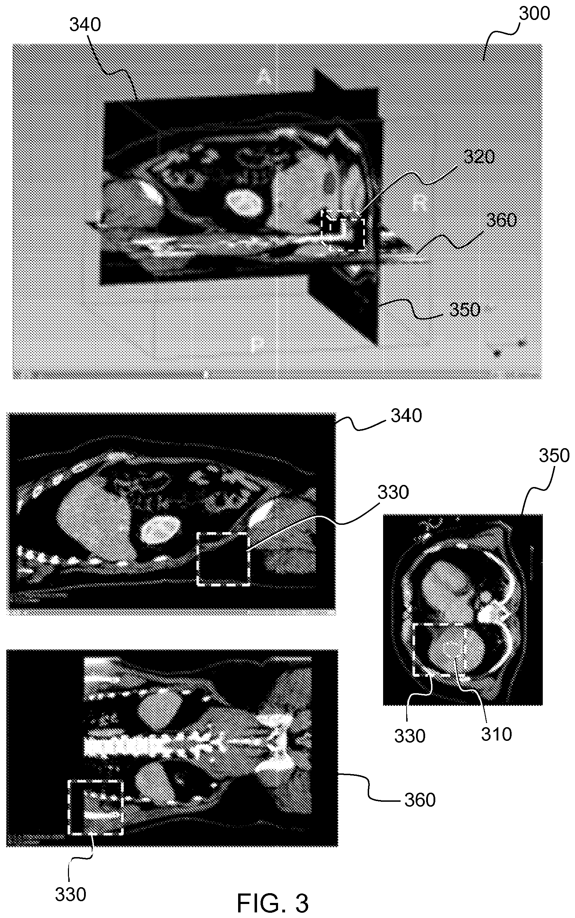

La figure 3 est une illustration d'une image médicale 300 en trois dimensions dans laquelle une région d'ablation 310 est entourée par un cadre 320. Le cadre 320 est cubique et correspond à des carrés 330 sur les vues en coupe 340, 350 et 360, respectivement selon la vue sagittale, selon la vue axiale et selon la vue coronale.

L'image médicale post-opératoire de l'anatomie d'intérêt 130 est ensuite analysée par un réseau de neurones, qui est une méthode d'apprentissage automatique, au cours d'une deuxième étape 220 afin de segmenter automatiquement la région d'ablation dans l'image médicale post-opératoire de l'anatomie d'intérêt 130 de l'individu 110.

À cet effet, le réseau de neurones a préalablement été entraîné sur une base de données d'images médicales d'une anatomie d'intérêt identique, ici par conséquent un foie, d'un ensemble de de patients au cours d'une phase préalable 290 d'entraînement. Chaque images médicale de la base de données comprend une anatomie d'intérêt ayant une fonction identique à celle de l'anatomie d'intérêt 130.

Avantageusement, l'image médicale post-opératoire de l'anatomie d'intérêt 130 de l'individu 110 est acquise dans la même modalité que celle des images médicales de la base de données d'entraînement du réseau de neurones.

Lorsque l'image médicale post-opératoire de l'anatomie d'intérêt 130 de l'individu 110 est recadrée, les dimensions du cube ou du carré de cette image médicale post-opératoire est avantageusement identique à celles des cubes ou carrés utilisés pour entraîner le réseau de neurones. En d'autres termes, les images médicales de la base de données ont les mêmes dimensions que l'image médicale post-opératoire recadrée.

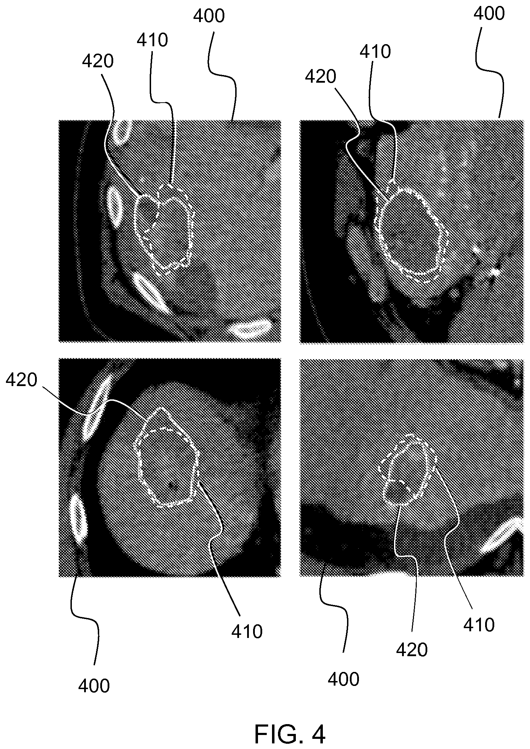

Afin d'entraîner le réseau de neurones, la région d'ablation de chaque image post-opératoire de la base de données, où la lésion a été ablatée, a été préalablement segmentée par au moins deux opérateurs, afin d'augmenter la pertinence de l'apprentissage et par conséquent des résultats d'analyse obtenus par le réseau de neurones. En effet, il peut être difficile pour un opérateur de délimiter une région d'ablation, notamment quand le contraste dans l'image est faible comme on peut le voir par exemple sur les quatre images médicales post-opératoires 400 préalablement segmentées de la figure 4. L'utilisation de plusieurs opérateurs annotant les images médicales permet donc d'améliorer l'identification de la région d'ablation. La région d'ablation associée à l'image post-opératoire recalée correspond ainsi dans le présent exemple non limitatif de l'invention à l'union des régions d'ablation proposées par les opérateurs. A titre d'exemple alternatif, la région d'ablation associée à l'image médicale post-opératoire recalée peut correspondre à l'intersection, à un consensus ou à une adjudication des régions d'ablation proposées par les opérateurs. Le réseau de neurones est en outre entrainé à classer les voxels d'une image médicale en région d'ablation ou de non-ablation.

Alternativement, l'apprentissage peut être effectué en utilisant un seul annotateur expert délimitant les régions d'ablation dans les images médicales. L'expérience de l'opérateur est alors importante afin que le réseau de neurones puisse aboutir à des régions d'ablation bien délimitées.

Il convient également de souligner qu'il est préférable que l'ensemble des images de la base de données comprenne autant de voxels appartenant à la région d'ablation que de voxels appartenant à une région de non-ablation. Cette proportion est calculée à partir de la classification des voxels déterminée manuellement par des opérateurs.

En d'autres termes, la partie du corps de l'individu comprise dans chaque image de la base de données est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

En outre, il est également préférable que la région d'ablation ne soit pas toujours au centre du cadre dans chaque image médicale de la base de données d'apprentissage. Dans le cas où la région d'ablation serait principalement au centre du cadre, un biais serait introduit dans le réseau de neurones qui apprendrait que la région d'ablation est principalement une région au centre du cadre, ce qui n'est pas nécessairement le cas, notamment en cas d'erreur de positionnement du cadre par un opérateur. Une variable aléatoire bornée est par conséquent avantageusement ajoutée à la position des cadres pour limiter ce biais de positionnement de la lésion au centre du cadre.

L'ensemble des centres de la région d'ablation des images médicales recadrées de la base de données forment ainsi une constellation de points distincts à l'intérieur du cadre commun.

Afin de limiter la production de faux positifs par le réseau de neurones, il est en outre préférable que la base de données comprenne des images médicales comprenant au moins une lésion non ablatée.

La phase 290 d'entraînement du réseau de neurones s'effectue généralement en plusieurs étapes :
- une étape 291 d'entraînement ;
- une étape 292 de validation ;
- une étape 293 de test.

La base de données d'images médicales est ainsi partitionnée en trois bases de données comprenant des images médicales distinctes. Les trois bases de données sont appelées respectivement base d'entrainement, base de validation et base de test. Dans le présent exemple non limitatif de l'invention, 60 à 98 % des images médicales de la base de données d'images médicales sont regroupées dans la base d'entraînement, 1 à 20 % dans la base de validation et 1 à 20 % dans la base de test. Les pourcentages, fonctions généralement du nombre d'images de la base de données d'images médicales, sont donnés ici à titre indicatif.

Les deux premières étapes 291 et 292 de la phase 290 d'entrainement du réseau de neurones sont des étapes principales pouvant être répétées plusieurs fois. La troisième étape de test est quant à elle optionnelle.

Lors de la première étape 291 de la phase 290 d'entrainement, un poids W et un biais b pour chaque neurone du réseau de neurones sont déterminés à partir des images médicales de la base d'entrainement.

Il convient de souligner que la base d'entrainement peut avantageusement comprendre des images médicales comprenant au moins une lésion non ablatée.

La deuxième étape 292 de la phase 290 d'entrainement permet de valider le poids W et le biais b déterminés préalablement pour chaque neurone du réseau de neurones, à partir des images médicales de la base de validation, afin de vérifier les résultats du réseau de neurones, notamment l'erreur de prédiction, c'est-à-dire en comparant pour image médicale de la base de données de validation la région d'ablation obtenue avec la région d'ablation segmentée dans l'image médicale extraite de la base d'entraînement.

Au cas où l'erreur de prédiction serait trop importante à l'issue de cette deuxième étape, les deux étapes d'entrainement 291 et de validation 292 sont mises en œuvre à nouveau pour réentraîner le réseau de neurones en réutilisant les mêmes images médicales, afin d'affiner les valeurs des poids W et des biais b de chaque neurone.

Alternativement, au cours du réentrainement du réseau de neurones, la première étape 291 utilise un rééchantillonnage des images médicales en considérant pour l'entrainement les images médicales de la base de données d'entrainement et une partie des images médicales de la base de validation. Le reste des images médicales de la base de validation est ensuite utilisé pour valider les poids W et les biais b obtenus à l'issu de la première étape du réentrainement.

Il convient de souligner que le réseau de neurones peut être réentraîné autant de fois que nécessaire jusqu'à que l'erreur de prédiction soit acceptable, c'est-à-dire soit inférieure à une valeur prédéterminée.

Lorsque les deux étapes 291 et 292 de la phase 290 d'entrainement sont mises en œuvre au moins une fois, les performances finales du réseau de neurones peuvent être testées au cours d'une éventuelle troisième étape 293 de test avec les images médicales de la base de test. Ces images médicales, distinctes des images médicales des bases d'entrainement et de validation, permettent de vérifier que le réseau de neurones tel que configuré avec les paramètres W et b pour chaque neurone permet de segmenter avec une bonne précision la région d'ablation dans toutes les situations auxquelles le réseau de neurones est susceptible d'être confronté. Toutefois, à la différence de l'étape 292 de validation, cette éventuelle troisième étape 293 de test n'aboutit pas à un nouveau cycle d'entrainement du réseau de neurones.

Il convient de souligner que les images utilisées lors de l'étape 293 de test sont généralement soigneusement sélectionnées afin de couvrir différentes positions et tailles de la région d'ablation dans l'anatomie d'intérêt afin de tester au mieux les capacités de prédiction du réseau de neurones.

A partir de l'image médicale post-opératoire de l'anatomie d'intérêt 130, le réseau de neurones classe chaque voxel en région d'ablation ou de non ablation. Cette prédiction peut prendre la forme d'un masque d'ablation superposé à l'image médicale post-opératoire de l'anatomie d'intérêt 130. Le masque d'ablation est généralement recalé sur les voxels appartenant à la région d'ablation prédite par le réseau de neurones. Il convient de souligner que le masque d'ablation est généralement délimité par une surface ou par un contour dans le cadre d'une image bidimensionnelle.

Avantageusement, avant la phase 290 d'entrainement le réseau de neurones peut avoir été préalablement entraîné, au cours d'une phase 295 dite de pré-entraînement, sur une deuxième base de données d'images médicales comprenant des images médicales présentant une lésion d'une anatomie d'intérêt de même type que celle de l'individu 110. Cette phase 295 de pré-entraînement permet d'obtenir une meilleure segmentation de la région d'ablation dans l'image médicale post-opératoire de l'individu 110, en utilisant astucieusement la similitude de forme d'une lésion et d'une région d'ablation, voire de position au sein de l'anatomie d'intérêt.

Il convient de souligner que les images médicales de la deuxième base de données peuvent avantageusement avoir été préalablement segmentées par au moins un opérateur. L'apprentissage lors de la phase 295 est similaire à celui effectué lors de la phase 290.

L'image médicale post-opératoire est recalée avec l'image médicale pré-opératoire 170 au cours d'une troisième étape 230 du procédé 200 illustré en figure 2. Le recalage permettant de retrouver les correspondances entre des points anatomiques des deux images médicales est effectué par une méthode connue de l'homme du métier. Le recalage peut être effectué de manière rigide, c'est-à-dire que tous les points des images sont transformés de la même manière, ou de manière non rigide, c'est-à-dire que chaque point des images peut avoir une transformation spécifique.

Une évaluation d'un risque de récidive est ensuite effectuée au cours d'une quatrième étape 240 comprenant quatre sous-étapes 241, 242, 243 et 244.

Au cours d'une éventuelle sous-étape 241, la lésion 165 est détectée dans l'image médicale pré-opératoire de l'anatomie d'intérêt 130 de l'individu 110. Cette détection peut être effectuée automatiquement ou manuellement par un opérateur.

Au cours de la sous-étape 242, une segmentation de la lésion 165 est effectuée automatiquement sur l'image médicale pré-opératoire de l'anatomie d'intérêt 130 de l'individu 110. Alternativement, la segmentation est effectuée manuellement par un opérateur.

La lésion est segmentée automatiquement par des méthodes connues de l'homme du métier. Par exemple, la segmentation est effectuée par une méthode basée sur l'histogramme de l'image, telle que par exemple la méthode d'Otsu, ou par une méthode d'apprentissage profond, plus couramment appelé par le terme anglais « *deep learning* ».

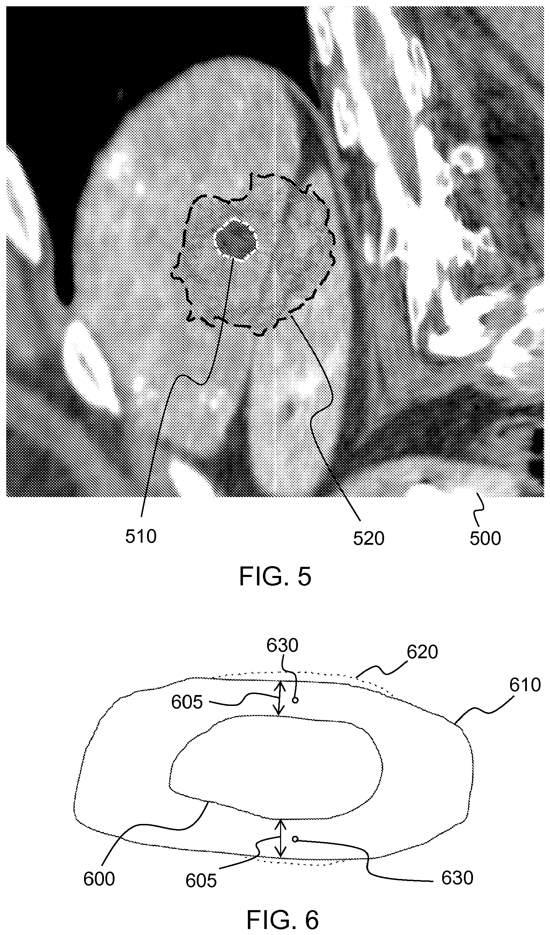

Cette sous-étape 242 de segmentation est illustrée par la figure 5 qui présente une image médicale 500 pré-opératoire dans laquelle une segmentation automatique basée sur une méthode d'apprentissage profond est effectuée pour déterminer l'emplacement tridimensionnel de la lésion 510 et de la région d'ablation 520. Un résultat équivalent peut être obtenu par un réseau de neurones distinct du réseau de neurones utilisé au cours de l'étape 230.

Une marge d'ablation est ensuite déterminée entre la segmentation de la lésion et le masque d'ablation préalablement établis, au cours de la sous-étape 243. La marge d'ablation correspond au minimum de marge, c'est-à-dire au minimum de distance, relevée entre la segmentation de la lésion et le masque d'ablation. En d'autres termes, la marge d'ablation correspond à la plus petite distance calculée entre un point de la lésion et un point de la région d'ablation et se calcule pour tous les points de la lésion.

La détermination des marges d'ablation permet de s'assurer que la région d'ablation recouvre bien la lésion.

À partir de la valeur de la marge d'ablation déterminée au cours de la sous-étape 243, une prédiction d'un risque de récidive voire une détermination de la position de la récidive peuvent être évaluées au cours de la sous-étape 244 en comparant la marge d'ablation calculée avec des valeurs de référence des marges d'ablations associées à un statut de récidive, stockées dans une base de données, le statut de récidive indiquant si une récidive a été constatée après l'opération ou non, avec éventuellement une date de récidive associée. Par exemple, il peut être considéré que le risque de récidive est nul lorsque les marges d'ablation sont égales ou supérieures à 5 mm.

Il convient de souligner que la prédiction d'un risque de récidive de la lésion prend généralement la forme d'une valeur binaire égale par exemple à 0 (risque nul ou négatif) ou 1 (risque avéré ou positif).

En complément ou de façon alternative, d'autres prédicteurs du risque de récidive que les marges d'ablation peuvent être utilisés. Le risque de récidive et la position de la récidive peuvent être estimés en pondérant tout ou partie ces différents prédicteurs. A titre d'exemple, les prédicteurs du risque de récidive sont basées sur des caractéristiques relatives pouvant être :
- une marge d'ablation ;
- une distance entre la surface de la lésion, ou une partie de la surface de la lésion, et la région d'ablation ;
- une distance entre les centres de masse de la lésion et les centres de masse de la région d'ablation ;
- une distance entre la surface de la lésion, ou une partie de la surface de la lésion et la région d'ablation et la distance entre les centres de masse de la lésion et les centres de masse de la région d'ablation en tenant compte de la proximité de la capsule de l'anatomie d'intérêt, notamment dans le cas des lésions sous-capsulaires ;
- une régularité des bords de la région d'ablation par rapport au tissu sain environnant ;
- une netteté des bords de la région d'ablation par rapport au tissu sain environnant ;
- un rapport entre le volume de la lésion et le volume de la région d'ablation ;
- une position de la lésion dans l'anatomie d'intérêt.

La valeur de référence du centre de masse dépend des marges d'ablation. Si les marges d'ablation sont égales à 10 mm et que la valeur de référence des marges d'ablation est de 5 mm, la distance entre les centres de masse de la lésion et les centres de masse de la région d'ablation doit être inférieure ou égale à 5 mm.

Il convient de souligner que la valeur du risque de récidive peut être avantageusement continu entre 0 et 1 au lieu d'être une valeur binaire, afin de prendre en considération plusieurs prédicteurs de risque. Une pondération peut ainsi être effectué entre différents prédicteurs de risque afin d'obtenir une valeur comprise entre 0 et 1. On entendra alors par le fait que le risque de récidive est avéré lorsqu'il est supérieur à une valeur seuil prédéterminée, par exemple égale à 0,5.

Dans le cas où le risque est avéré, une détermination de la position de la récidive peut être effectuée afin d'estimer un masque d'ablation complémentaire au cours d'une cinquième étape 250 du procédé 200 d'évaluation post-traitement. Le maque d'ablation complémentaire est par exemple considéré pour effectuer une ablation complémentaire dans un région où la marge d'ablation est inférieure à une valeur donnée, par exemple cinq millimètres.

Au cours de l'étape 250, une identification automatique d'une région où la marge d'ablation est inférieure à une valeur seuil, par exemple inférieure à cinq millimètres peut être effectuée.

La figure 6 illustre un exemple d'ablation complémentaire suite à un traitement d'ablation d'une lésion 600 avec risque de récidive. Le procédé 200 d'évaluation a identifié des régions dans lesquelles les marges d'ablation 605 étaient insuffisantes entre la lésion 600 et la région d'ablation 610, et a généré un masque 620 d'ablation complémentaire. Il convient de souligner que la génération du masque 620 d'ablation complémentaire est effectuée en tentant de limiter au maximum la zone d'ablation. En outre, des points cibles 630 à atteindre par une aiguille d'ablation peuvent alors être définis dans le masque 620.

Le procédé 200 peut également comprendre une étape 260 de planification d'une trajectoire à suivre par l'instrument médical 120 associée soit au masque d'ablation ou au masque d'ablation complémentaire, afin de guider l'opérateur lors de la manipulation de l'instrument médical 120 au cours d'une étape 270 de guidage de l'instrument médical 120 selon la trajectoire planifiée.

Un exemple de méthode de planification est décrit dans la demande de brevet français n°1914780 intitulée « *Méthode de planification automatique d'une trajectoire pour une intervention médicale* ».

Il convient de souligner que le guidage est dans le présent exemple non limitatif de l'invention du type visuel en affichant la trajectoire planifiée et/ou d'une indication de guidage sur l'écran 155 du casque 150.

Alternativement, le guidage de l'instrument médical 120 peut être effectué par l'intermédiaire d'un système de navigation fournissant des informations de position et d'orientation de l'instrument médical 120. Il peut s'agir d'un guidage mécanique par l'intermédiaire d'un dispositif robotisé couplé à un tel système de navigation.

Il convient de souligner que les étapes 230 à 260 peuvent être répétées jusqu'à ce que le risque de récidive soit nul ou quasiment nul, ou jusqu'à ce que les marges d'ablation soient suffisantes.

## Revendications

1. Procédé (200) d'évaluation post-traitement d'une ablation d'une partie d'une anatomie d'intérêt (130) d'un individu (110), l'anatomie d'intérêt comprenant au moins une lésion (165, 510, 600), l'ablation de la partie de l'anatomie d'intérêt étant délimitée par une région d'ablation (160), le procédé d'évaluation comprennant des étapes de :
• acquisition (210) d'une image médicale post-opératoire de l'anatomie d'intérêt de l'individu, comprenant tout ou partie de la région d'ablation ;
• détermination automatique d'un contour de la région d'ablation par une méthode d'apprentissage automatique, de type réseau de neurones, analysant l'image post-traitement de l'anatomie d'intérêt de l'individu, ladite méthode d'apprentissage automatique étant préalablement entraînée lors d'une phase (290) dite d'entraînement sur une base de données comportant une pluralité d'images médicales post-opératoire d'une anatomie d'intérêt identique d'un ensemble de patients, chaque image médicale de la base de données étant associée à une région d'ablation de l'anatomie d'intérêt dudit patient.
**caractérisé en ce que** la phase d'entraînement comprend une étape préalable d'entraînement sur des images médicales d'une anatomie d'intérêt identique comprenant une lésion non ablatée.

2. Procédé d'évaluation post-traitement selon l'une quelconque des revendications précédentes, comprenant également une étape de recalage (220) de l'image post-opératoire et d'une image médicale (170, 500) de l'anatomie d'intérêt de l'individu, acquise avant le traitement chirurgical, dite image médicale pré-opératoire.

3. Procédé d'évaluation post-traitement selon l'une quelconque des revendications précédentes, comprenant également une étape d'évaluation d'un risque de récidive en fonction d'une caractéristique relative entre la région d'ablation et la lésion, entre la région d'ablation et l'anatomie d'intérêt ou entre la lésion et l'anatomie d'intérêt.

4. Procédé d'évaluation post-traitement selon la revendication précédente, comprenant également, lorsque le risque de récidive est avéré, une étape de détermination de la position de la récidive en fonction d'une caractéristique relative entre la région d'ablation et la lésion, entre la région d'ablation et l'anatomie d'intérêt ou entre la lésion et l'anatomie d'intérêt.

5. Procédé d'évaluation post-traitement selon l'une quelconque des revendications 3à 4, dans lequel une caractéristique relative est une marge d'ablation entre la région d'ablation et la lésion.

6. Procédé d'évaluation post-traitement selon l'une quelconque des revendications 3 à 5, dans lequel une caractéristique relative est un centre de masse de la lésion et un centre de masse de la région d'ablation.

7. Procédé d'évaluation post-traitement selon l'une quelconque des revendications 3 à 6, dans lequel une caractéristique relative est la régularité et de la netteté des bords de la région d'ablation par rapport au tissu sain environnant.

8. Procédé d'évaluation post-traitement selon l'une quelconque des revendications 3 à 7, dans lequel une caractéristique relative est le rapport entre le volume de la lésion et le volume de la région d'ablation.

9. Procédé d'évaluation post-traitement selon l'une quelconque des revendications 3 à 8, dans lequel une caractéristique relative est une position de la lésion par rapport au centre de l'anatomie d'intérêt.

10. Procédé d'évaluation post-traitement selon l'une quelconque des revendications 2 à 9, comprenant également une étape de segmentation de la lésion dans l'image médicale pré-opératoire de l'anatomie d'intérêt de l'individu.

11. Procédé d'évaluation post-traitement selon l'une quelconque des revendications 2 à 10, comprenant également une étape de détection de la lésion dans l'image médicale pré-opératoire de l'anatomie d'intérêt de l'individu.

12. Procédé d'évaluation post-traitement selon l'une quelconque des revendications 2 à 11, dans lequel tout ou partie des images médicales post-opératoires d'entrainement de la base de données sont recadrées, autour de la région d'ablation comprenant au moins une lésion, le recadrage des images étant effectué selon un cadre commun de dimension prédéterminée, l'ensemble des centres de la région d'ablation des images médicales post-opératoires recadrées formant une constellation de points distincts à l'intérieur du cadre commun.

13. Procédé d'évaluation post-traitement selon l'une quelconque des revendications 2 à 12, dans lequel pour l'ensemble des images médicales post-opératoires de la base de données, la partie du corps de l'individu comprise dans l'image est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

14. Procédé d'évaluation post-traitement selon l'une quelconque des revendications 2 à 13, dans lequel la base de données d'images médicales post-opératoires comprend au moins une image médicale pré-opératoire comprenant au moins une lésion non ablatée.

15. Procédé d'évaluation post-traitement selon l'une quelconque des revendications 3 à 14, comprenant également une étape de détermination d'un masque d'ablation complémentaire lorsque le risque de récidive est avéré.

16. Procédé d'évaluation post-traitement selon la revendication précédente, comprenant également une étape de proposition d'une trajectoire à suivre par un instrument médical jusqu'à un point cible du masque d'ablation complémentaire.

17. Procédé d'évaluation post-traitement selon l'une quelconque des revendications précédentes, dans lequel les images médicales sont des images tridimensionnelles.

18. Procédé d'évaluation post-traitement selon l'une quelconque des revendications 2 à 17, dans lequel chaque image post-opératoire est acquise selon une même technique d'acquisition d'image.

19. Dispositif (150) électronique comprenant un processeur et une mémoire informatique stockant des instructions d'un procédé d'évaluation post-traitement selon l'une quelconque des revendications précédentes.

20. Dispositif électronique selon la revendication précédente, pouvant être un dispositif de contrôle, un système de navigation, un dispositif robotisé ou un dispositif de réalité augmentée.

## Patentansprüche

1. Verfahren (200) zur Nachbehandlungsbewertung einer Ablation eines Teils einer Anatomie (130) von Interesse einer Person (110), wobei die Anatomie von Interesse mindestens eine Läsion (165, 510, 600) umfasst, wobei die Ablation des Teils der Anatomie von Interesse durch einen Ablationsbereich (160) begrenzt ist, wobei das Bewertungsverfahren folgende Schritte umfasst:
• Erfassen (210) eines postoperativen medizinischen Bildes der Anatomie von Interesse der Person, das den gesamten oder einen Teil des Ablationsbereichs umfasst;
• automatisches Bestimmen einer Kontur des Ablationsbereichs durch ein maschinelles Lernverfahren, vom Typ neuronales Netzwerk, durch Analyse des Nachbehandlungsbildes der Anatomie von Interesse der Person, wobei das maschinelle Lernverfahren zuvor während einer Trainingsphase (290) auf einer Datenbank trainiert wird, die eine Vielzahl von postoperativen medizinischen Bildern einer identischen Anatomie von Interesse einer Gruppe von Patienten aufweist, wobei jedes medizinische Bild in der Datenbank mit einem Ablationsbereich der Anatomie von Interesse des Patienten verknüpft ist,
**dadurch gekennzeichnet, dass** die Trainingsphase einen vorangehenden Trainingsschritt anhand medizinischer Bilder einer identischen Anatomie von Interesse umfasst, die eine nicht abladierte Läsion umfasst.

2. Verfahren zur Nachbehandlungsbewertung nach einem der vorhergehenden Ansprüche, ferner umfassend einen Schritt (220) der Registrierung des postoperativen Bildes und eines medizinischen Bildes (170, 500) der Anatomie von Interesse der Person, das vor der chirurgischen Behandlung erfasst wurde, dem präoperativen medizinischen Bild.

3. Verfahren zur Nachbehandlungsbewertung nach einem der vorhergehenden Ansprüche, ferner umfassend einen Schritt des Bewertens eines Rezidivrisikos basierend auf einem relativen Merkmal zwischen dem Ablationsbereich und der Läsion, zwischen dem Ablationsbereich und der Anatomie von Interesse oder zwischen der Läsion und der Anatomie von Interesse.

4. Verfahren zur Nachbehandlungsbewertung nach dem vorhergehenden Anspruch, ferner umfassend, wenn das Risiko eines Rezidivs nachgewiesen ist, einen Schritt des Bestimmens der Position des Rezidivs basierend auf einem relativen Merkmal zwischen dem Ablationsbereich und der Läsion, zwischen dem Ablationsbereich und der Anatomie von Interesse oder zwischen der Läsion und der Anatomie von Interesse.

5. Verfahren zur Nachbehandlungsbewertung nach einem der Ansprüche 3 bis 4, wobei ein relatives Merkmal ein Ablationsrand zwischen dem Ablationsbereich und der Läsion ist.

6. Verfahren zur Nachbehandlungsbewertung nach einem der Ansprüche 3 bis 5, wobei ein relatives Merkmal ein Massenzentrum der Läsion und ein Massenzentrum des Ablationsbereichs ist.

7. Verfahren zur Nachbehandlungsbewertung nach einem der Ansprüche 3 bis 6, wobei ein relatives Merkmal die Regelmäßigkeit und Schärfe der Ränder des Ablationsbereichs in Bezug auf das umgebende gesunde Gewebe ist.

8. Verfahren zur Nachbehandlungsbewertung nach einem der Ansprüche 3 bis 7, wobei ein relatives Merkmal das Verhältnis zwischen dem Volumen der Läsion und dem Volumen des Ablationsbereichs ist.

9. Verfahren zur Nachbehandlungsbewertung nach einem der Ansprüche 3 bis 8, wobei ein relatives Merkmal eine Position der Läsion in Bezug auf das Zentrum der Anatomie von Interesse ist.

10. Verfahren zur Nachbehandlungsbewertung nach einem der Ansprüche 2 bis 9, ferner umfassend einen Schritt des Segmentierens der Läsion in dem präoperative medizinische Bild der Anatomie von Interesse der Person.

11. Verfahren zur Nachbehandlungsbewertung nach einem der Ansprüche 2 bis 10, ferner umfassend einen Schritt des Erkennens der Läsion in dem präoperativen medizinischen Bild der Anatomie von Interesse der Person.

12. Verfahren zur Nachbehandlungsbewertung nach einem der Ansprüche 2 bis 11, wobei die postoperativen medizinischen Trainingsbilder der Datenbank ganz oder teilweise um den Ablationsbereich, der mindestens eine Läsion umfasst, zugeschnitten werden, wobei das Zuschneiden der Bilder nach einem gemeinsamen Rahmen vorbestimmter Größe durchgeführt wird, wobei die Gesamtheit der Zentren des Ablationsbereichs der zugeschnittenen postoperativen medizinischen Bilder eine Konstellation von getrennten Punkten innerhalb des gemeinsamen Rahmens bilden.

13. Verfahren zur Nachbehandlungsbewertung nach einem der Ansprüche 2 bis 12, wobei für alle postoperativen medizinischen Bilder der Datenbank der in dem Bild umfasste Körperteil der Person in eine Vielzahl von Elementareinheiten einer Größe unterteilt ist, wobei die Anzahl der Elementareinheiten in nahezu gleiche Teile zwischen dem durch den Ablationsbereich begrenzten Teil des menschlichen Körpers und dem restlichen Teil des Körpers der Person aufgeteilt wird, der in dem Bild umfasst ist.

14. Verfahren zur Nachbehandlungsbewertung nach einem der Ansprüche 2 bis 13, wobei die Datenbank mit postoperativen medizinischen Bildern mindestens ein präoperatives medizinisches Bild umfasst, das mindestens eine nicht abladierte Läsion umfasst.

15. Verfahren zur Nachbehandlungsbewertung nach einem der Ansprüche 3 bis 14, ferner umfassend einen Schritt des Bestimmens einer zusätzlichen Ablationsmaske, wenn das Risiko eines Rezidivs nachgewiesen ist.

16. Verfahren zur Nachbehandlungsbewertung nach dem vorhergehenden Anspruch, ferner umfassend einen Schritt des Vorschlagens einer Trajektorie, die von einem medizinischen Instrument bis zu einem Zielpunkt der zusätzlichen Ablationsmaske verfolgt werden soll.

17. Verfahren zur Nachbehandlungsbewertung nach einem der vorhergehenden Ansprüche, wobei die medizinischen Bilder dreidimensionale Bilder sind.

18. Verfahren zur Nachbehandlungsbewertung nach einem der Ansprüche 2 bis 17, wobei jedes postoperative Bild nach demselben Bilderfassungsverfahren erfasst wird.

19. Elektronische Vorrichtung (150), die einen Prozessor und einen Computerspeicher zum Speichern von Anweisungen eines Verfahrens zur Nachbehandlungsbewertung nach einem der vorhergehenden Ansprüche umfasst.

20. Elektronische Vorrichtung nach dem vorhergehenden Anspruch, die eine Kontrollvorrichtung, ein Navigationssystem, eine Robotervorrichtung oder eine Augmented-Reality-Vorrichtung sein kann.

## Claims

1. Method (200) for the post-treatment evaluation of an ablation of a portion of an anatomical structure of interest (130) of an individual (110), the anatomical structure of interest comprising at least one lesion (165, 510, 600), the ablation of the portion of the anatomical structure of interest being delimited by an ablation region (160), the evaluation method comprising the steps of:
• acquiring (210) a post-operative medical image of the anatomical structure of interest of the individual, comprising all or part of the ablation region;
• automatically determining a contour of the ablation region using a machine learning method of the neural network type, analysing the post-treatment image of the anatomical structure of interest of the individual, said machine learning method being previously trained in a phase (290), referred to as a training phase, using a database comprising a plurality of post-operative medical images of an identical anatomical structure of interest from a set of patients, each medical image in the database being associated with an ablation region of the anatomical structure of interest of said patient.
**characterised in that** the training phase comprises a prior step of training using medical images of an identical anatomical structure of interest comprising a non-ablated lesion.

2. Post-treatment evaluation method according to the preceding claim, further comprising a step of registering (220) the post-operative image and a medical image (170, 500) of the anatomical structure of interest of the individual acquired before the surgical treatment, referred to as a pre-operative medical image.

3. Post-treatment evaluation method according to any one of the preceding claims, further comprising a step of evaluating a risk of recurrence based on a relative characteristic between the ablation region and the lesion, between the ablation region and the anatomical structure of interest or between the lesion and the anatomical structure of interest.

4. Post-treatment evaluation method according to the preceding claim, further comprising, when the risk of recurrence is demonstrated, a step of determining the position of the recurrence according to a relative characteristic between the ablation region and the lesion, between the ablation region and the anatomical structure of interest or between the lesion and the anatomical structure of interest.

5. Post-treatment evaluation method according to any one of Claims 3 to 4, wherein a relative characteristic is an ablation margin between the ablation region and the lesion.

6. Post-treatment evaluation method according to any one of Claims 3 to 5, wherein a relative characteristic is a centre of mass of the lesion and a centre of mass of the ablation region.

7. Post-treatment evaluation method according to any one of Claims 3 to 6, wherein a relative characteristic is the regularity and sharpness of the edges of the ablation region in relation to the surrounding healthy tissue.

8. Post-treatment evaluation method according to any one of Claims 3 to 7, wherein a relative characteristic is the ratio of the volume of the lesion to the volume of the ablation region.

9. Post-treatment evaluation method according to any one of Claims 3 to 8, wherein a relative characteristic is a position of the lesion in relation to the centre of the anatomical structure of interest.

10. Post-treatment evaluation method according to any one of Claims 2 to 9, further comprising a step of segmenting the lesion in the pre-operative medical image of the anatomical structure of interest of the individual.

11. Post-treatment evaluation method according to any one of Claims 2 to 10, further comprising a step of detecting the lesion in the pre-operative medical image of the anatomical structure of interest of the individual.

12. Post-treatment evaluation method according to any one of Claims 2 to 11, wherein all or some of the training post-operative medical images of the database are cropped around the ablation region comprising at least one lesion, the cropping of the images being carried out using a common frame of predetermined size, the set of the centres of the ablation region in the cropped post-operative medical images forming a constellation of distinct points inside the common frame.

13. Post-treatment evaluation method according to any one of Claims 2 to 12, wherein for the set of post-operative medical images in the database, the part of the individual's body included in the image is divided into a plurality of elementary units of a single size, the number of elementary units being divided into almost equal parts between the part of the human body delimited by the ablation region and the rest of the part of the individual's body included in the image.

14. Post-treatment evaluation method according to any one of Claims 2 to 13, wherein the post-operative medical image database comprises at least one pre-operative medical image comprising at least one non-ablated lesion.

15. Post-treatment evaluation method according to any one of Claims 3 to 14, further comprising a step of determining a supplementary ablation mask when the risk of recurrence is demonstrated.

16. Post-treatment evaluation method according to the preceding claim, further comprising a step of proposing a path to be followed by a medical instrument to a target point of the supplementary ablation mask.

17. Post-treatment evaluation method according to any one of the preceding claims, wherein the medical images are three-dimensional images.

18. Post-treatment evaluation method according to any one of Claims 2 to 17, wherein each post-operative image is acquired using the same image acquisition technique.

19. Electronic device (150) comprising a processor and a computer memory storing instructions of a post-treatment evaluation method according to any one of the preceding claims.

20. Electronic device according to the preceding claim, which may be a control device, a navigation system, a robotic device or an augmented reality device.
